# FASCICULE DE BREVET EUROPEEN

(11) **EP 3 481 504 B1**
(45) Date de publication et mention de la délivrance du brevet: **06.08.2025**
(21) Numéro de dépôt: 17737551.6
(22) Date de dépôt: 06.07.2017
(51) Int. Cl.: A61N 7/00, A61B 18/00

(54) **PROCÉDÉ ET SYSTÈME POUR LA DÉTECTION D'UN DÉFAUT DE RACCORDEMENT ÉLECTRIQUE ENTRE UN DISPOSITIF ULTRASONORE ET UNE UNITÉ DE COMMANDE DISTANTE**
VERFAHREN UND SYSTEM ZUR ERKENNUNG EINES FEHLERS BEI EINER ELEKTRISCHEN VERBINDUNG ZWISCHEN EINER ULTRASCHALLVORRICHTUNG UND EINER FERNBEDIENUNGSEINHEIT
METHOD AND SYSTEM FOR DETECTING A FAULT IN AN ELECTRICAL CONNECTION BETWEEN AN ULTRASOUND DEVICE AND A REMOTE CONTROL UNIT

(30) Priorité: 08.07.2016 FR 1656611
(43) Date de publication de la demande: 15.05.2019
(73) Titulaire: Carthera, 75013 Paris (FR)
(72) Inventeur: CANNEY, Michael, Denver, Colorado 80210 (US)
(74) Mandataire: Be IP
(86) Numéro de dépôt international: PCT/EP2017/066899
(87) Numéro de publication internationale: WO 2018/007500

(56) Documents cités:
- EP-A2- 2 539 021
- US-A- 4 791 915
- US-A1- 2003 028 341
- US-A1- 2012 083 717
- US-A1- 2014 171 802
- US-A1- 2015 157 299

## Description

### DOMAINE DE L'INVENTION

La présente invention concerne le domaine technique général des dispositifs ultrasonore - par exemple des dispositifs intracorporels ou implantables - destinés à être raccordés électriquement à une unité de commande distante.

De tels dispositifs peuvent notamment être implantés chez l'homme et les mammifères pour aider un praticien dans l'établissement d'un diagnostic et/ou pour traiter une pathologie.

### ARRIERE PLAN DE L'INVENTION

On connaît du document EP 2 539 021 un appareil de traitement d'affections du cerveau. En référence à la figure 1, un tel appareil se compose :
- d'un dispositif ultrasonore 1 réalisé en matériau non ferromagnétique,
- d'une unité de commande 2 distante du dispositif ultrasonore 1, et
- de moyens de connexion entre le dispositif ultrasonore 1 et l'unité de commande 2.

Le dispositif ultrasonore 1 est destiné à être positionné dans un trou de trépan effectué dans un crâne d'un patient. Il est avantageusement compatible avec la technique d'Imagerie par Résonance Magnétique (IRM), et comprend :
- un boîtier 11 composé de parois constituées dans un matériau isolant électriquement,
- au moins un transducteur 12 positionné dans le boîtier pour la génération d'ondes ultrasonores de traitement d'une affection cérébrale,
- des moyens de fixation 13 pour fixer le boitier 11 dans le crâne du patient,
- une (ou plusieurs) borne(s) 14 de connexion électrique destinée(s) à coopérer avec les moyens de connexion.

Les moyens de connexion sont destinés à relier électriquement le dispositif ultrasonore 1 à l'unité de commande 2. Ils comprennent généralement :
- un (ou plusieurs) câble(s) 31 de connexion électrique dont l'une des extrémités est reliée à l'unité de commande, et
- une (ou plusieurs) aiguille(s) transdermique(s) 32 raccordée(s) à l'autre extrémité du câble 31.

Le principe de fonctionnement de cet appareil est le suivant. Une fois le dispositif ultrasonore 1 implanté dans le crâne du patient, une succession de séances de traitement lui sont prodiguées pour traiter la pathologie qui l'affecte. A chaque nouvelle séance de traitement, le dispositif intracorporel 1 est relié à l'unité de commande 2 par l'intermédiaire des moyens de connexion.

Le praticien relie le câble 31 à l'unité de commande 2 puis il insère l'aiguille 32 à travers la peau du patient jusqu'à la borne 14 du dispositif ultrasonore.

Une fois l'extrémité de l'aiguille 32 connectée à la borne 14, l'unité de commande 2 peut être activée pour alimenter le dispositif ultrasonore 1 en énergie électrique.

Même si l'appareil décrit dans EP 2 539 021 permet un traitement efficace des affections du cerveau, il n'existe pas à l'heure actuelle de technique permettant d'informer le praticien d'un éventuel défaut du raccordement électrique entre le dispositif intracorporel et l'unité de commande 2. Un tel défaut de raccordement électrique peut engendrer :
- des problèmes de sécurité pour le patient (brûlures, choc électrique, etc.), et/ou
- des problèmes d'efficacité du dispositif ultrasonore concernant le diagnostic et/ou le traitement d'une pathologie.

Le document US 2014/171802 décrit un dispositif de diagnostic comprenant :
- une sonde ultrasonore incluant une mémoire et une unité de génération d'état binarisé pour générer un état électrique binarisé,
- une unité de conversion de données d'identification de sonde qui convertit l'état électronique en données d'identification de sonde,
- une unité de lecture qui lit des informations d'identification de sonde provenant de la mémoire,
- une unité de comparaison qui détermine une conformité entre les données d'identification et des informations d'identification de sonde lues, et
- une unité de sortie d'alerte qui, si les données d'identification et les informations d'identification de sonde ne sont pas conformes, émet un signal d'alerte.

Le document US 4 791 915 décrit un appareil de thérapie par ultrasons comportant un transducteur portatif connecté à une unité de base dotée d'un écran avant et d'un panneau de commande. Parmi les affichages du panneau se trouve un graphique à barres représentant un pourcentage de couplage.

Le document US 2015/157299 décrit un système à ultrasons incluant un réseau de transducteurs et un processeur configuré pour détecter les éléments sous performants du réseau de transducteurs.

Le document US 2003/028341 décrit un système d'imagerie par ultrasons comprenant un autotest à la mise sous tension (POST) et un autotest étendu (EST). Ces autotests POST et EST peuvent consister en un code source enregistré dans une mémoire du système d'imagerie, et être exécutés par un processeur du système d'imagerie.

Le document US 2012/083717 décrit un système et un procédé pour une sonothrombolyse transcranienne non invasive d'une vascularisation cérébrale ciblée qui assure un positionnement optimisé en une seule étape de transducteurs ultrasonores par rapport à la cible sans avoir besoin d'un positionnement réitératif et/ou d'une rétroaction représentant la qualité d'un traitement aux ultrasons.

Le document EP 2 539 021 décrit un appareil de traitement d'une tumeur cérébrale.

Un but de la présente invention est de proposer un procédé et un système permettant au praticien de détecter un éventuel défaut de raccordement électrique entre :
- un dispositif intracorporel 1 implanté dans un patient et
- une unité de commande 2 externe.

### BREVE DESCRIPTION DE L'INVENTION

A cet effet, l'invention propose un appareil d'aide au diagnostic et/ou de traitement d'une pathologie selon la revendication 1.

On entend, dans le cadre de la présente invention, par *« défaut de raccordement électrique »*, une imperfection dans la connexion électrique entre le dispositif de génération d'ultrasons et la sonde, cette imperfection empêchant la circulation d'un courant électrique entre le dispositif de génération d'ultrasons et la sonde. En d'autres termes un *« défaut de raccordement »* consiste en l'absence de liaison électrique entre le dispositif de génération d'ultrasons et la sonde.

Des aspects préférés mais non limitatifs de l'appareil d'aide sont définis aux revendications 2 à 7.

L'invention concerne également un procédé de détection d'un défaut fonctionnement tel que défini à la revendication 8.

Des aspects préférés mais non limitatifs du procédé selon l'invention sont définis aux revendications 9 à 11.

### BREVE DESCRIPTION DES DESSINS

D'autres avantages et caractéristiques du procédé selon l'invention ressortiront mieux de la description qui va suivre de plusieurs variantes d'exécution, données à titre d'exemples non limitatifs, à partir des dessins annexés sur lesquels :
- La figure 1 illustre schématiquement un exemple d'appareil de traitement d'une infection cérébrale incluant un dispositif ultrasonore raccordé électriquement à une unité de commande distante grâce à des moyens de connexion (aiguille transdermique + câble),
- La figure 2 illustre des étapes d'un procédé de détection d'un défaut de raccordement électrique entre le dispositif et l'unité de commande,
- La figure 3 illustre une première variante de réalisation du procédé de détection de la figure 2,
- La figure 4 illustre un premier exemple de stratégie de détection selon la première variante de réalisation du procédé,
- La figure 5 illustre une deuxième variante du procédé de détection de la figure 2,
- La figure 6 illustre schématiquement un exemple d'unité de commande pour la mise en œuvre du procédé selon l'invention,
- la figure 7 est une courbe de tension en fonction du temps.

### DESCRIPTION DETAILLEE DE L'INVENTION

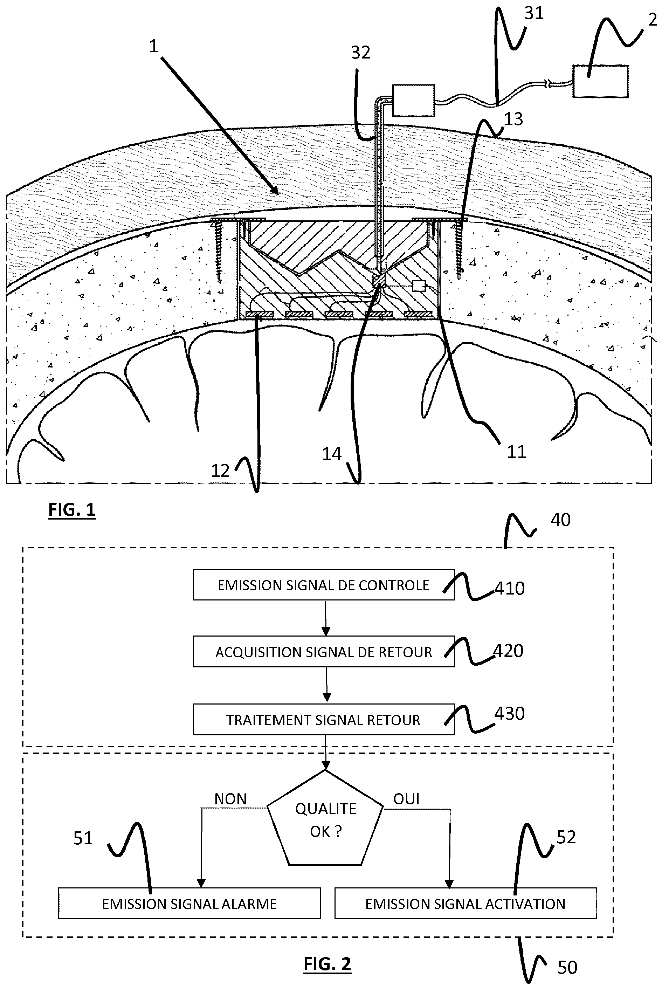

On va maintenant décrire différents exemples de procédé de détection en référence aux figures 2 à 7. Dans ces différentes figures, les éléments équivalents sont désignés par la même référence numérique.

Ce procédé de détection permet à un praticien de vérifier si le raccordement électrique entre une unité de commande externe et un dispositif ultrasonore implanté dans le corps d'un patient est correctement réalisé.

Dans la suite, on décrira le procédé de détection en référence à l'appareil présenté dans le document EP 2 539 021.

Toutefois, il est bien évident pour l'homme du métier que le procédé selon l'invention peut être mis en œuvre avec tout type d'appareil de traitement incluant un dispositif ultrasonore - dispositif intracorporel, dispositif implantable ou dispositif non-implantable
- devant être raccordé électriquement à une unité de commande distante.

Comme décrit précédemment, l'appareil comprend :
- un dispositif ultrasonore 1 comprenant un boîtier 11 dans lequel est logé au moins un transducteur 12 pour la génération d'ondes ultrasonores,
- une unité de commande 2 distante pour alimenter en énergie électrique le dispositif ultrasonore 1, et régler ses paramètres de fonctionnement,
- des moyens de connexion (aiguille transdermique + câble) pour raccorder électriquement le dispositif ultrasonore 1 et l'unité de commande 2.

Cet appareil permet le traitement d'une affection cérébrale en mettant en œuvre plusieurs séances de traitement prescrites par le praticien, chaque séance se composant d'une succession de cycles d'activation précédés chacun d'un cycle d'attente.

Durant un cycle d'attente, le dispositif ultrasonore 1 est désactivé pendant une durée d'attente (de l'ordre de 975 millisecondes). Cette désactivation est réalisée en n'alimentant pas le dispositif ultrasonore 1 en énergie électrique.

Lorsque la durée d'attente est expirée, un cycle d'activation est mis en œuvre. L'activation du dispositif ultrasonore 1 est réalisée en alimentant celui-ci avec une énergie électrique pendant une durée d'activation (de l'ordre de 25 millisecondes). Cette énergie électrique est avantageusement émise par l'unité de commande 2 à une fréquence de travail du transducteur 12. Le transducteur 12 génère des ondes ultrasonores en direction de la zone cérébrale située juste en dessous du dispositif ultrasonore 1.

Lorsque la durée d'activation est expirée, un nouveau cycle d'attente est mis en œuvre, et ainsi de suite jusqu'à la fin de la séance.

Le procédé de détection décrit dans la suite propose d'utiliser le cycle d'attente précédant chaque cycle d'activation pour détecter la qualité du raccordement électrique entre le dispositif ultrasonore 1 et l'unité de commande 2.

### 1. Procédé de détection de la qualité d'un raccordement électrique entre le dispositif ultrasonore et l'unité de commande

On va maintenant décrire plus en détails différentes variantes de réalisation du procédé de détection.

On suppose dans la suite que le dispositif ultrasonore 1 a été implanté dans le crâne du patient et que le praticien a raccordé électriquement le dispositif ultrasonore 1 à l'unité de commande2.

En référence à la figure 2, le procédé de détection comprend les étapes suivantes :
- Pendant chaque cycle d'attente 40 :
   o L'émission 410 par l'unité de commande 2 d'au moins un signal de contrôle à un premier instant du cycle d'attente 40,
   o L'acquisition 420 par l'unité de commande 2 d'au moins un signal de retour à un deuxième instant du cycle d'attente 40,
   o Le traitement 430 du signal de retour pour obtenir une information sur la qualité du raccordement électrique entre le dispositif ultrasonore 1 et l'unité de commande 2,
- Pendant chaque cycle d'activation 50, l'émission 51, 52 d'un signal en fonction de l'information obtenue sur la qualité du raccordement électrique, ledit signal consistant en :
   ∘ un signal d'activation si l'unité de commande 2 est correctement raccordée au dispositif ultrasonore 1,
   ∘ un signal d'alarme si l'unité de commande 2 n'est pas correctement raccordée au dispositif ultrasonore 1.

Chaque signal de contrôle est émis à une faible énergie électrique par rapport au signal d'activation (de l'ordre de 1% de l'énergie requise pour le traitement). Ceci permet d'éviter les risques d'échauffement du patient lors de la phase de contrôle de la qualité du raccordement électrique entre le dispositif ultrasonore 1 et l'unité de commande 2, notamment dans le cas d'un raccordement défectueux. Notamment, dans l'hypothèse d'un court-circuit au niveau de l'aiguille transdermique par contact de ses pôles avec un tissu, l'émission du signal d'activation peut provoquer un choc électrique. Même si celui-ci n'est pas nécessairement dangereux, il peut être douloureux pour le patient et occasionner la brûlure d'une petite zone de tissu dans le cuir chevelu.

Ainsi, le procédé selon l'invention permet au praticien de vérifier la qualité du raccordement électrique entre le dispositif ultrasonore 1 et l'unité de commande 2 préalablement à chaque phase d'activation. On garantit ainsi l'efficacité du traitement lors de chaque cycle d'activation.

Différents types de raccordement défectueux peuvent être rencontrés :
- Le câble 31 peut ne pas être relié électriquement à l'unité de commande 2,
- L'aiguille transdermique 32 peut ne pas être reliée électriquement à la borne 14 ; dans ce cas, la pointe de l'aiguille 32 est en contact :
   o soit avec un isolant électrique (aiguille 32 n'ayant pas traversé la couche de matériau isolant recouvrant la borne 14),
   o soit avec un conducteur électrique (peau du crâne ou fluide circulant dans le crâne du patient).

Les deux variantes du procédé décrites dans la suite permettent de détecter ces différents types de raccordement électrique défectueux.

### 1.1. Premier mode de réalisation

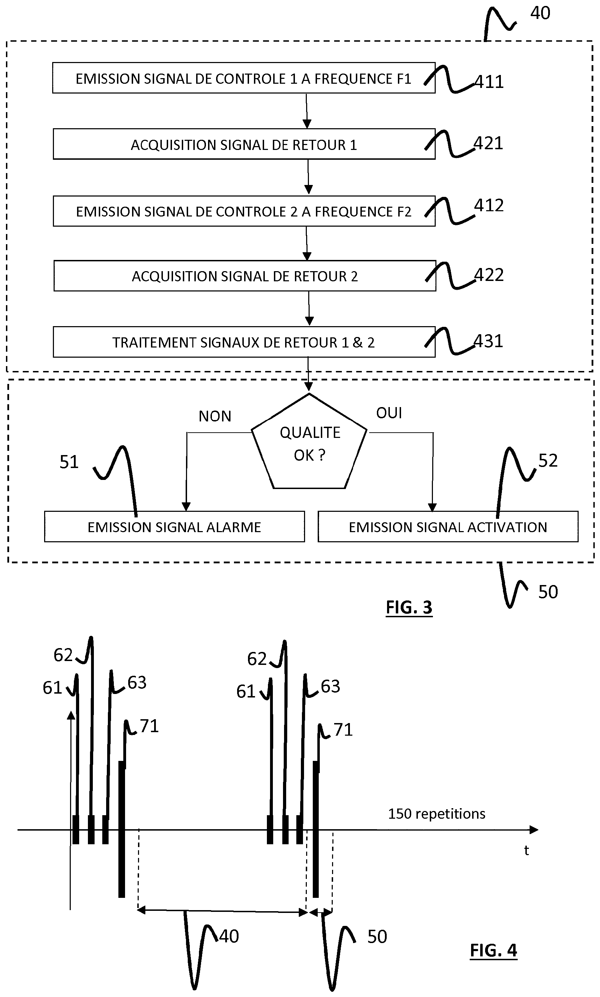

Dans une première variante de réalisation illustrée aux figures 3 et 4, le procédé utilise une approche multi-fréquentielle. En particulier dans cette première variante, des signaux de contrôle sont émis à deux fréquences distinctes.

Le fait d'émettre des signaux de contrôle à des fréquences différentes permet d'améliorer la fiabilité du procédé de détection des raccordements électriques défectueux.

En effet, si une seule fréquence est utilisée avec une tolérance sur la puissance réfléchie, alors :
- la puissance réfléchie dans le cas où l'aiguille 32 est correctement reliée au dispositif ultrasonore 1, et
- la puissance réfléchie dans le cas où la pointe de l'aiguille 32 est en contact avec un tissu (qui est conducteur)
peuvent être semblables, de sorte qu'il est difficile de différencier un raccordement électrique correct d'un raccordement électrique défectueux dans lequel l'aiguille est simplement dans un milieu conducteur (les deux pôles qui sont situés à la pointe de l'aiguille étant alors en court-circuit).

L'approche multifréquences - i.e. l'utilisation de signaux de contrôle à deux fréquences distinctes - permet de lever cette ambiguïté. En effet, le dispositif ultrasonore a la particularité de présenter une impédance variant en fonction de la fréquence du signal électrique qui lui est appliqué.

Avantageusement, les signaux de contrôle émis lors du cycle d'attente 40 sont des courants électriques impulsionnels, les fréquences choisies pour l'émission desdits signaux de contrôle étant :
- Une première fréquence F1 (par exemple de l'ordre de 1,05 MHz) choisie dans une plage de fréquence de travail - tel qu'une fréquence de résonance - du transducteur 12,
- Une deuxième fréquence F2 distincte de la première fréquence F1 choisie en dehors de la plage de fréquences de travail de travail du transducteur 12, de sorte que la majorité du signal de contrôle émis à la fréquence F2 par l'unité de commande 2 soit réfléchi vers celle-ci.

En référence à la figure 3, le procédé peut comprendre les étapes suivantes :
- Pendant le cycle d'attente 40 :
   ∘ émission 411 d'un premier signal de contrôle impulsionnel basse puissance à une première fréquence F1 choisie dans une plage de fréquences de travail du transducteur 12 du dispositif ultrasonore 1, et acquisition 421 d'un premier signal de retour en réponse,
   ∘ émission 412 d'un deuxième signal de contrôle impulsionnel basse puissance à une deuxième fréquence F2 choisie en dehors de la plage de fréquence de travail du transducteur 12, et acquisition 422 d'un deuxième signal de retour en réponse,
   ∘ traitement 431 des premier et deuxième signaux de retour pour détecter un éventuel défaut de raccordement,
- Pendant le cycle d'activation 50 :
   ∘ émission 51 d'un signal d'alarme si un défaut de raccordement est détecté,
   ∘ émission 52 d'un signal d'activation sinon, le signal d'activation consistant en un signal électrique impulsionnel haute puissance émis à la fréquence de travail F1 du transducteur 12.

Avantageusement, l'acquisition des premier et deuxième signaux de retour peut consister à mesurer les puissances électriques desdits signaux de retour, par exemple en utilisant un coupleur directionnel. Ceci permet de limiter la complexité de l'unité de commande.

Outre les défauts de raccordement électrique entre le dispositif ultrasonore 1 et l'unité de commande 2, l'approche multifréquences peut permettre de détecter les défauts de fabrication de l'aiguille transdermique 32, tel qu'un court-circuit au niveau des pôles de l'aiguille 32, par exemple en mettant en œuvre le procédé illustré à la figure 3 préalablement à l'insertion de l'aiguille 32 dans le patient.

La figure 4 illustre un exemple de stratégie pouvant être employée pour détecter un défaut de raccordement en mettant en œuvre la première variante du procédé selon l'invention.

Trois signaux de contrôle 61, 62, 63 - consistant chacun en une impulsion électrique de basse puissance et d'une durée de 100 microsecondes - sont émis vers le dispositif ultrasonore pendant chaque cycle d'attente 40 :
- une première impulsion 61 émise à la fréquence F2 choisie en dehors de la plage de fréquences de travail du transducteur 12,
- une deuxième impulsion 62 émise à la fréquence F1 choisie dans la plage de fréquence de travail du transducteur 12,
- une troisième impulsion 63 émise à la fréquence F2.

Des premier, deuxième et troisième signaux de retour sont acquis en réponse à l'émission des première, deuxième et troisième impulsions 61, 62, 63.

Ces premier, deuxième et troisième signaux de retour sont comparés à des valeurs seuil contenues dans une mémoire de l'unité de commande 2. Cette comparaison permet de déterminer si l'unité de commande 2 est correctement raccordée au dispositif ultrasonore 1, ou s'il existe un défaut dans le raccordement électrique.

Si l'unité de commande 2 est correctement raccordée au dispositif ultrasonore 1, un signal d'activation 71 à la fréquence F1, de haute puissance (i.e. de puissance supérieur à la puissance des signaux de contrôle) et d'une durée de 23.8 microsecondes est émis vers le dispositif ultrasonore 1 pendant chaque cycle d'activation 50. L'émission du signal d'activation 71 induit la génération d'ondes ultrasonores permettant le traitement du patient.

Les cycles d'attente et d'activation 40, 50 sont ensuite répétés une pluralité de fois (150 fois dans l'exemple illustré à la figure 4).

Le tableau ci-dessous illustre comment la comparaison de deux signaux de retour P_{R1}, P_{R2} (acquis en réponse à l'émission de deux signaux de contrôle émis à des fréquences F1 et F2) avec des valeurs seuils S₁, S₂ permet de déterminer si le raccordement électrique entre le dispositif ultrasonore 1 et l'unité de commande 2 est correct ou non.

Le raccordement électrique est considéré comme correct si des première et deuxième conditions (relatives à la comparaison des signaux de retour P_{R1}, P_{R2} aux valeurs seuils S₁, S₂) sont satisfaites en combinaison :
- la première condition sur le signal de retour P_{R1} est satisfaite si la puissance électrique mesurée est supérieure à la valeur seuil S₁,
- la deuxième condition sur le signal de retour P_{R2} est satisfaite si la puissance électrique mesurée est inférieure à la valeur seuil S₂.

Si l'une et/ou l'autre des première et deuxième conditions n'est (ne sont) pas satisfaite(s), alors le raccordement électrique entre l'unité de commande et le dispositif ultrasonore est défectueuse.

| | Conditions | F2 : 0.6 MHz | F1 : 1.05 MHz |
|---|---|---|---|
| | | S₂ = 100 | S₁ = 210 |
| 1 | Raccordement à l'unité de commande : **NON** | P_{R2} = 24 | P_{R1} = 57 |
| | Raccordement à l'implant : **NON** | P_{R2} < S₂ | P_{R1} < S₁ |
| | | **=> OK** | => **ALARME** |
| 2 | Raccordement à l'unité de commande : **OUI** | P_{R2} = 30 | P_{R1} = 63 |
| | Raccordement à l'implant : **NON** | P_{R2} < S₂ | P_{R1} < S₁ |
| | Pointe de l'aiguille dans milieu isolant | **=> OK** | => **ALARME** |
| 3 | Raccordement à l'unité de commande : **OUI** | P_{R2}= 107 | P_{R1} = 193 |
| | Raccordement à l'implant : **NON** | P_{R2} > S₂ | P_{R1} < S₁ |
| | Pointe de l'aiguille dans milieu conducteur | => **ALARME** | => **ALARME** |
| 4 | Raccordement à l'unité de commande : **OUI** | P_{R2} = 79 | P_{R1} = 257 |
| | Raccordement à l'implant : **OUI** | P_{R2} < S₂ | P_{R1} > S₁ |
| | Transducteur en contact acoustique avec milieu conducteur ou le tissu | **=> OK** | => **OK** |
| 5 | Raccordement à l'unité de commande : **OUI** | P_{R2} = 57 | P_{R1} = 212 |
| | Raccordement à l'implant : **OUI** | P_{R2} < S₂ | P_{R1} > S₁ |
| | Transducteur en l'air, n'est PAS en contact acoustique avec milieu conducteur ou le tissu | => **OK** | => **OK** |

Le cas n° 5 est représentatif d'une situation dans laquelle le raccordement électrique entre le dispositif ultrasonore 1 et l'unité de commande 2 est correct, mais où le transducteur 12 n'est pas en contact avec le tissu à traiter. L'homme du métier appréciera que l'ajout d'une troisième condition (associée à la comparaison du signal de retour P_{R1} à une troisième valeur seuil S₃ (par exemple égale à 230) pourrait permettre de détecter cette anomalie. Le lecteur appréciera que les valeurs P_{R1}, P_{R2} correspondent à des valeurs brutes obtenues à partir d'un convertisseur analogique-numérique.

Dans le cadre de l'exemple ci-dessus, celle-ci n'ont pas été converties en valeurs de puissance, et les seuils S₁, S₂ mentionnés ci-dessus correspondent à une échelle arbitraire des niveaux de puissance.

Bien entendu les valeurs P_{R1}, P_{R2} pourraient être converties en valeurs de puissance, par exemple en utilisant une fonction quadratique ou polynomiale déterminée par un processus d'étalonnage de l'appareil selon l'invention.

### 1.2. Deuxième mode de réalisation

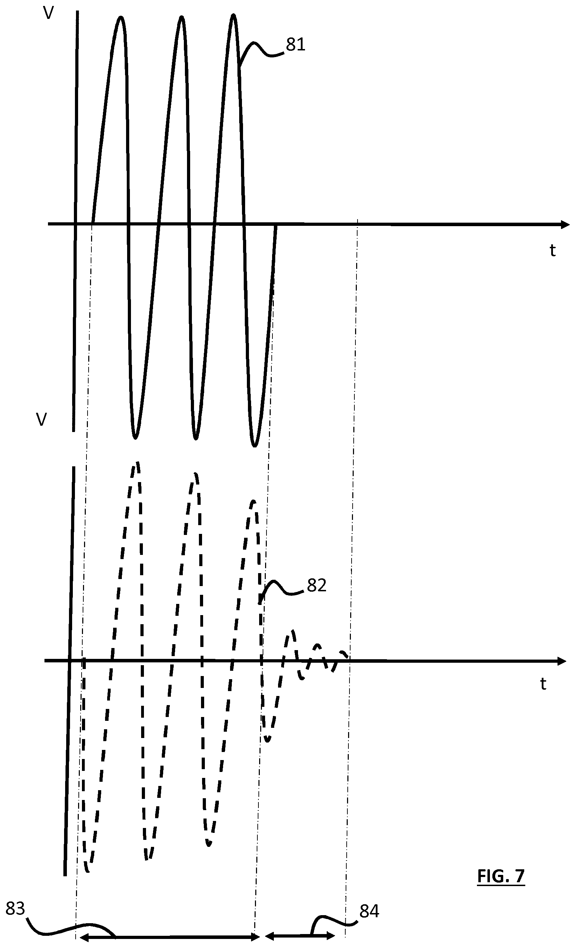

Dans une deuxième variante de réalisation, le procédé utilise une approche vibratoire. L'approche vibratoire est basée sur le fait que le transducteur 12 est un élément résonant. Lorsqu'un tel élément résonant est excité par une impulsion électrique émise à une fréquence choisie dans sa plage de travail, il continue à vibrer même après la fin de l'excitation. La figure 7 illustre ce phénomène dans le cas d'un transducteur 12 excité par un signal d'activation 81 pendant une durée d'activation 83. On constate du signal réfléchi 82 par le transducteur 12 que celui-ci continue à vibrer pendant une durée non nulle 84 après la fin de l'excitation.

Cette vibration « résiduelle » peut être mesurée en utilisant plusieurs techniques :
- soit en mesurant directement la tension envoyée au transducteur 12, ce qui nécessite que le signal de tension soit numérisé à une fréquence supérieure ou égale à deux fois la fréquence d'excitation (limite de Nyquist) ; cette technique nécessite l'intégration d'un numériseur à haute vitesse dans l'unité de commande,
- soit en mesurant la tension réfléchie par le transducteur 12 ; cette technique nécessite l'intégration d'un convertisseur de valeur quadratique moyenne (ou « RMS » sigle de l'expression anglo-saxonne « Root Mean Square ») ou d'un détecteur de pics permettant d'identifier des pics de la tension réfléchie mais permet de s'affranchir de l'utilisation d'un numériseur à haute vitesse.

La signature du signal de retour est unique au dispositif ultrasonore 1 et ne se produit pas lorsque l'aiguille 32 est placée dans un matériau conducteur tel qu'une solution saline.

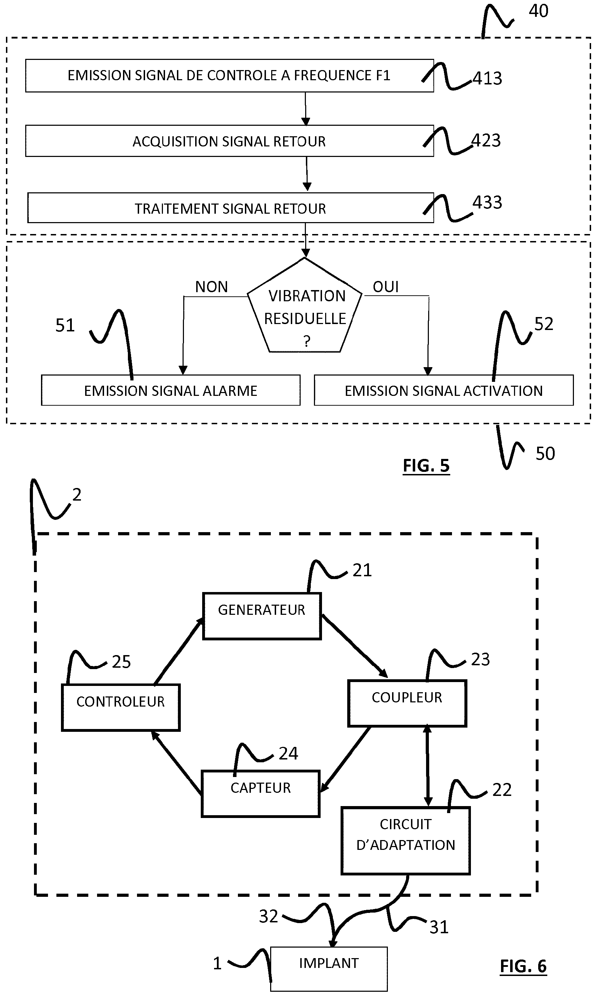

En référence à la figure 5, le procédé peut comprendre les étapes suivantes :
- Pendant chaque cycle d'attente 40 :
   ∘ émission 413 d'un signal de contrôle impulsionnel basse puissance à une fréquence F1 choisie dans une plage de fréquences de travail du transducteur 12,
   ∘ acquisition 423 d'un signal de retour en réponse,
   ∘ traitement 433 du signal de retour pour détecter un éventuel défaut de raccordement, le traitement consistant à extraire les pics du signal de retour après la fin d'émission du signal de contrôle afin d'en déduire l'état (vibratoire ou non vibratoire) du transducteur 12,
- Pendant chaque cycle d'activation 50 :
   ∘ émission 51 d'un signal d'alarme si un défaut de raccordement est détecté,
   ∘ émission 52 d'un signal d'activation sinon, le signal d'activation consistant en un signal électrique impulsionnel haute puissance émis à la fréquence F1 du transducteur 12.

### 2. Unité de commande

En référence à la figure 6, on a illustré l'unité de commande 2 d'un exemple d'appareil pour la mise en œuvre du procédé décrit précédemment.

L'unité de commande 2 comprend :
- un générateur 21 d'alimentation en énergie électrique pour fournir de l'énergie électrique au dispositif ultrasonore,
- un circuit d'adaptation d'impédance 22 pour optimiser le transfert d'énergie électrique entre le générateur et le dispositif ultrasonore,
- un coupleur bidirectionnel 23 entre le générateur 21 et le circuit d'adaptation d'impédance 22,
- un capteur 24 en aval du coupleur pour extraire des signaux reçus du coupleur 23 une valeur de puissance électrique,
- un contrôleur 25 pour traiter les signaux issus du capteur 24 et informer le praticien de l'état du raccordement électrique entre le dispositif ultrasonore 1 et l'unité de commande 2.

Le coupleur bidirectionnel 23 permet d'acquérir les signaux de retour. Plus précisément, le coupleur 23 permet de mesurer les signaux réfléchis par le dispositif ultrasonore 1, et les moyens de connexion (câble 31 /aiguille 32). Le coupleur bidirectionnel 23 est par exemple le modèle ZFBDC20-61HP+ de la société Mini-Circuits^{®}, utilisé en combinaison d'un convertisseur analogique numérique à bas coût tel que le modèle Picoscope 3206B de la société Pico-Technology^{®}. Avantageusement, le coupleur bidirectionnel 23 est positionné en amont du circuit d'adaptation d'impédance 22 ; ceci permet de simplifier le traitement des signaux de retour pour en extraire la puissance électrique réfléchie.

Le principe de fonctionnement de l'unité de commande 2 est le suivant. Pendant un (ou chaque) cycle d'attente 40, le contrôleur 25 commande au générateur 21 l'émission d'un signal de contrôle basse puissance. Le signal de contrôle généré par le générateur 21 traverse le coupleur bidirectionnel 23 et le circuit d'adaptation d'impédance 22. Il est émis vers le dispositif ultrasonore 1 par l'intermédiaire des moyens de connexion électrique (câble 31 + aiguille 32).

Le coupleur bidirectionnel 23 acquiert un signal de retour (ou plusieurs signaux de retour). Plus précisément, le coupleur bidirectionnel 23 mesure le signal radiofréquence réfléchi par le dispositif intra ultrasonore corporel 1, les moyens de connexion 31, 32, ou l'absence de tel signal radiofréquence.

Le signal de retour acquis par le coupleur bidirectionnel 23 est transmis au capteur 24 qui le traite pour en extraire une valeur de puissance électrique. Cette valeur de puissance électrique est transmise au contrôleur 25 qui la compare à une (ou plusieurs) valeur(s) seuil pour détecter un éventuel défaut de raccordement électrique.

Si aucun défaut de raccordement n'est détecté, le contrôleur 25 commande au générateur 21 l'émission d'un signal d'activation haute puissance à la fréquence F1 pour induire la génération d'ondes ultrasonores par le transducteur 12 du dispositif ultrasonore 1.

Si un défaut de raccordement est détecté, le contrôleur 25 émet un signal d'alerte afin d'avertir le praticien dudit défaut, par exemple en émettant un stimulus sonore et/ou visuel sur une interface de l'unité de commande 2 (l'interface pouvant comprendre un écran et/ou une enceinte).

Ainsi, la présente invention propose une solution au problème de la détection des défauts de raccordement entre un dispositif ultrasonore implantable et une unité de commande distante. En effet le dispositif permet de détecter une *« coupure »* dans le circuit de connexion.

La présente invention permet également, comme décrit précédemment de détecter un défaut de couplage acoustique entre l'appareil de traitement (ou d'imagerie) et le tissu à traiter (ou imager), par exemple en détectant une variation d'impédance du (ou des) transducteur(s). Cette variation d'impédance peut être due à un défaut de contact acoustique entre le transducteur et le tissu. Une variation d'impédance du transducteur peut aussi résulter d'un défaut du transducteur lui-même, par exemple un court-circuit ou un circuit ouvert. Lorsque la présente invention permet de détecter à la fois un défaut de raccordement électrique et un défaut de couplage acoustique, le procédé peut comprendre les étapes suivantes :
- Pendant un cycle d'attente :
   o L'émission par l'unité de commande d'au moins un signal de contrôle à un premier instant du cycle d'attente,
   o L'acquisition par l'unité de commande d'au moins un signal de retour à un deuxième instant du cycle d'attente,
   o Le traitement du signal de retour pour obtenir une information sur la qualité du raccordement électrique entre le dispositif ultrasonore et l'unité de commande et sur la qualité du couplage acoustique entre l'appareil et le tissu,
- Pendant un cycle d'activation (50) postérieur au cycle d'attente, l'émission d'un signal en fonction de l'information obtenue sur la qualité du raccordement électrique et de l'information obtenue sur la qualité du couplage acoustique, ledit signal consistant en :
   ∘ un signal d'activation si l'unité de commande est correctement raccordée au dispositif ultrasonore et si l'appareil est correctement couplé au tissu,
   ∘ un signal d'alarme si l'unité de commande n'est pas correctement raccordée au dispositif ultrasonore ou si l'appareil n'est pas correctement couplé au tissu.

Le lecteur aura compris que de nombreuses modifications peuvent être apportées à l'invention décrite précédemment sans sortir matériellement des nouveaux enseignements et des avantages décrits ici.

Par exemple, le procédé selon l'invention peut être utilisé avec d'autres appareils de traitement que celui décrit dans le document EP 2 539 021.

Egalement dans la description qui précède, le circuit d'adaptation d'impédance a été décrit comme étant intégré à l'unité de commande. En variante, le circuit d'adaptation d'impédance peut être intégré au dispositif ultrasonore.

De plus, d'autres signaux électriques (de contrôle ou d'alimentation du dispositif ultrasonore) peuvent être émis par l'unité de commande lors du cycle d'attente. Par exemple, dans une variante de réalisation de l'invention, le dispositif ultrasonore comprend un démultiplexeur connecté à une pluralité de transducteurs. Ce démultiplexeur permet l'activation séquentielle des transducteurs (ou l'activation simultanée de certains transducteurs sélectionnés parmi la pluralité de transducteurs). Dans ce cas, l'unité de contrôle peut être programmée pour interroger le démultiplexeur pendant un (ou les) cycles d'attente afin de vérifier qu'il répond (communication numérique bidirectionnel). A cet effet, le procédé peut comprendre les étapes supplémentaires suivantes :
- Pendant un (ou chaque) cycle d'attente :
   o émission par l'unité de commande d'une requête de communication vers le dispositif de génération d'ultrasons,
   o acquisition par l'unité de commande d'un message de réponse émis par le dispositif de génération d'ultrasons,
- Pendant le cycle d'activation :
   o émission d'un signal d'alarme si aucun message de réponse n'a été acquis.

Ces étapes supplémentaires peuvent être mises en œuvre préalablement, ou simultanément aux étapes relatives à la détection d'un défaut de raccordement électrique.

## Revendications

1. Appareil d'aide au diagnostic et/ou de traitement d'une pathologie comprenant :
- un dispositif de génération d'ultrasons (1) préalablement implanté,
- une unité de commande (2) distante pour déterminer et contrôler des paramètres de fonctionnement du dispositif (1), et lui délivrer de l'électricité pendant au moins un cycle d'activation (50), chaque cycle d'activation (50) étant précédé d'un cycle d'attente (40),
- des moyens de connexion électrique (31, 32) entre le dispositif (1) et l'unité de commande (2),
***caractérisé en ce que*** l'unité de commande (2) est programmée pour détecter, pendant au moins un cycle d'attente (40), un défaut du raccordement électrique consistant en une absence de liaison électrique entre le dispositif (1) et ladite unité de commande (2), ladite détection d'un défaut de raccordement consistant en :
- Pendant ledit et au moins un cycle d'attente (40) :
∘ l'émission (410, 411, 412, 413) par l'unité de contrôle d'au moins un signal de contrôle à au moins un premier instant du cycle d'attente, ledit et au moins un signal de contrôle comprenant un signal électrique impulsionnel basse puissance émis à une fréquence F1 choisie dans une plage de fréquences de travail d'un transducteur (12) du dispositif (1),
o l'acquisition (420, 421, 422, 423) par l'unité de contrôle d'au moins un signal de retour en réponse audit et au moins un signal de contrôle émis,
o le traitement (430, 431, 433) par l'unité de contrôle dudit et au moins un signal de retour acquis pour détecter un défaut de raccordement électrique entre le dispositif (1) et l'unité de commande (2),
- Pendant ledit et au moins un cycle d'activation (50) postérieur audit et au moins un cycle d'attente (40) :
o l'émission (52) par l'unité de contrôle, d'un signal d'activation du dispositif (1) si aucun défaut de raccordement électrique n'est détecté,
o l'absence d'émission par l'unité de contrôle, du signal d'activation si un défaut de raccordement électrique est détecté.

2. Appareil selon la revendication 1, dans lequel l'impédance du dispositif (1) varie en fonction de la fréquence du signal de contrôle.

3. Appareil selon l'une quelconque des revendications 1 ou 2, dans lequel ledit et au moins un signal de contrôle comprend :
- un premier signal de contrôle consistant en un signal électrique impulsionnel émis à la première fréquence F1 choisie dans la plage de fréquences de travail du transducteur (12) du dispositif (1),
- un deuxième signal de contrôle consistant en un signal-électrique impulsionnel émis à une deuxième fréquence F2 choisie en dehors de la plage de fréquences de travail du transducteur (12).

4. Appareil selon la revendication 3, dans lequel ledit et au moins un signal de retour comprend un premier signal de retour acquis en réponse à l'émission du premier signal de contrôle, et un deuxième signal de retour acquis en réponse à l'émission du deuxième signal de contrôle, l'unité de commande (2) étant programmée pour :
- Pendant ledit et au moins un cycle d'attente (40) :
o Comparer (431) les premier et deuxième signaux de retour à des première et deuxième valeurs seuil pour détecter un défaut de raccordement électrique entre le dispositif (1) et l'unité de commande (2),
- Pendant ledit et au moins un cycle d'activation (50) consécutif audit et au moins un cycle d'attente (40) :
∘ Emettre (52) vers le dispositif (1) un signal d'activation si aucun défaut de raccordement électrique n'est détecté, le signal d'activation consistant en un signal électrique impulsionnel émis à la première fréquence F1 choisie dans la plage de fréquences de travail du transducteur (12), la puissance électrique du signal d'activation étant supérieure à la puissance électrique des signaux de contrôle,
∘ ne pas émettre le signal d'activation sinon.

5. Appareil selon l'une quelconque des revendications 1 à 4, dans lequel l'étape de traitement comprend une sous-étape consistant à comparer la puissance électrique de chaque signal de retour à au moins une valeur seuil.

6. Appareil selon la revendication 1, dans lequel l'unité de commande (2) est programmée pour :
- Pendant ledit et au moins un cycle d'attente (40) :
o traiter (433) ledit et au moins un signal de retour pour déterminer un état vibratoire ou non vibratoire du transducteur (12) afin d'en déduire un éventuel défaut de raccordement,
- Pendant ledit et au moins un cycle d'activation (50) consécutif audit et au moins un cycle d'attente (40) :
∘ émettre (52) vers le dispositif (1) le signal d'activation si aucun défaut de raccordement électrique n'est détecté, le signal d'activation consistant en un signal électrique impulsionnel émis à la première fréquence F1 choisie dans la plage de fréquences de travail du transducteur (12), la puissance électrique du signal d'activation étant supérieure à la puissance électrique des signaux de contrôle,
∘ ne pas émettre le signal d'activation sinon.

7. Appareil selon l'une quelconque des revendications 1 à 6, dans lequel l'unité de commande (2) comprend un coupleur directionnel (23) pour l'acquisition dudit et au moins un signal de retour, ledit coupleur directionnel étant relié en amont d'un circuit d'adaptation d'impédance (22).

8. Procédé de détection d'un défaut de fonctionnement d'un appareil d'aide au diagnostic et/ou de traitement d'une pathologie par application d'ultrasons sur un tissu, l'appareil comprenant :
- un dispositif de génération d'ultrasons (1) préalablement implanté,
- une unité de commande (2) distante pour délivrer de l'électricité au dispositif (1) et déterminer et contrôler ses paramètres de fonctionnement, l'unité de commande (2) étant adaptée pour délivrer de l'électricité au dispositif (1) pendant au moins un cycle d'activation (50) de sorte à activer ledit dispositif (1), chaque cycle d'activation (50) étant précédé d'un cycle d'attente (40), le dispositif (1) et l'unité de commande (2) étant raccordés électriquement par l'intermédiaire de moyens de connexion électrique (31, 32),
**caractérisé en ce que** le procédé comprend une phase de contrôle mise en œuvre pendant au moins un cycle d'attente (40) pour détecter un défaut du raccordement électrique consistant en une absence de liaison électrique entre le dispositif (1) et l'unité de commande (2), ladite phase de contrôle comprenant les étapes suivantes :
- Pendant ledit et au moins un cycle d'attente (40) :
∘ L'émission (410) par l'unité de commande (2) d'au moins un signal de contrôle à un premier instant du cycle d'attente (40), ledit et au moins un signal de contrôle consistant en un signal électrique impulsionnel basse puissance émis à une fréquence (F1) choisie dans une plage de fréquences de travail d'un transducteur (12) du dispositif (1),
∘ L'acquisition (420) par l'unité de commande (2) d'au moins un signal de retour à un deuxième instant du cycle d'attente (40),
∘ Le traitement (430) dudit et au moins un signal de retour pour obtenir une information sur la qualité du raccordement électrique entre le dispositif ultrasonore (1) et l'unité de commande (2),
- Pendant ledit et au moins un cycle d'activation (50) postérieur audit et au moins un cycle d'attente (40), l'émission (51, 52) d'un signal en fonction de l'information obtenue sur la qualité du raccordement électrique, ledit signal consistant en :
∘ un signal d'alarme si l'unité de commande (2) n'est pas correctement raccordée au dispositif ultrasonore (1).

9. Procédé selon la revendication 8, dans lequel ledit et au moins un signal de contrôle comprend :
- un premier signal de contrôle consistant en un signal électrique impulsionnel émis à la première fréquence (F1) choisie dans la plage de fréquences de travail du transducteur (12) du dispositif (1),
- un deuxième signal de contrôle consistant en un signal électrique impulsionnel émis à une deuxième fréquence (F2) choisie en dehors de la plage de fréquences de travail du transducteur (12).

10. Procédé selon la revendication 9, lequel comprend les étapes suivantes :
- Pendant ledit et au moins un cycle d'attente (40) :
∘ émission (411) par l'unité de commande du premier signal de contrôle, et acquisition (421) par l'unité de commande d'un premier signal de retour en réponse,
∘ émission (412) par l'unité de commande du deuxième signal de contrôle impulsionnel, et acquisition (422) par l'unité de commande d'un deuxième signal de retour en réponse,
∘ traitement (431) des premier et deuxième signaux de retour pour détecter un éventuel défaut de raccordement.

11. Procédé selon la revendication 8, lequel comprend en outre une phase de surveillance mise en œuvre pendant au moins un cycle d'attente (40) pour détecter un défaut de couplage acoustique entre le dispositif de génération d'ultrasons et le tissu.

## Patentansprüche

1. Apparat zur Unterstützung der Diagnose und/oder der Behandlung einer Erkrankung, umfassend:
- eine zuvor implantierte Ultraschallerzeugungsvorrichtung (1),
- eine entfernte Steuereinheit (2) zum Bestimmen und Überwachen der Betriebsparameter der Vorrichtung (1) und zum Versorgen derselben mit Strom während mindestens eines Aktivierungszyklus (50), wobei jedem Aktivierungszyklus (50) ein Wartezyklus (40) vorausgeht,
- elektrische Anschlussmittel (31, 32) zwischen der Vorrichtung (1) und der Steuereinheit (2),
**dadurch gekennzeichnet, dass** die Steuereinheit (2) programmiert ist, um während mindestens eines Wartezyklus (40) einen elektrischen Anschlussfehler zu erkennen, der aus einer fehlenden elektrischen Verbindung zwischen der Vorrichtung (1) und der Steuereinheit (2) besteht, wobei die Erkennung eines Anschlussfehlers besteht aus:
- während des und mindestens eines Wartezyklus (40):
∘ dem Senden (410, 411, 412, 413) mindestens eines Prüfsignals durch die Steuereinheit zu mindestens einem ersten Zeitpunkt des Wartezyklus (40), wobei das und mindestens eine Prüfsignal ein niederfrequentes elektrisches Impulssignal umfasst, das mit einer Frequenz F1 gesendet wird, die aus einem Arbeitsfrequenzbereich eines Wandlers (12) der Vorrichtung (1) ausgewählt ist,
∘ dem Erfassen (420, 421, 422, 423) mindestens eines Rücksignals durch die Steuereinheit als Antwort an das und mindestens eine gesendete Prüfsignal,
∘ dem Verarbeiten (430, 431, 433) des und mindestens eines erfassten Rücksignals durch die Steuereinheit, um einen elektrischen Anschlussfehler zwischen der Vorrichtung (1) und der Steuereinheit (2) zu erkennen,
- während des und mindestens eines Aktivierungszyklus (50) nach dem und mindestens einem Wartezyklus (40):
∘ dem Senden (52) eines Aktivierungssignals der Vorrichtung (1) durch die Steuereinheit, wenn kein elektrischer Anschlussfehler erkannt wird,
∘ dem Nicht-Senden des Aktivierungssignals durch die Steuereinheit, wenn ein elektrischer Anschlussfehler erkannt wird.

2. Apparat nach Anspruch 1, wobei die Impedanz der Vorrichtung (1) in Abhängigkeit von der Frequenz des Prüfsignals schwankt.

3. Apparat nach einem der Ansprüche 1 oder 2, wobei das und mindestens eine Prüfsignal umfasst:
- ein erstes Prüfsignal, das aus einem elektrischen Impulssignal besteht, das mit der Frequenz (F1) gesendet wird, die aus dem Arbeitsfrequenzbereich des Wandlers (12) der Vorrichtung (1) ausgewählt ist,
- ein zweites Prüfsignal, das aus einem elektrischen Impulssignal besteht, das mit einer zweiten Frequenz F2 gesendet wird, die außerhalb des Arbeitsfrequenzbereichs des Wandlers (12) ausgewählt ist.

4. Apparat nach Anspruch 3, wobei das und mindestens eine Rücksignal ein erstes Rücksignal umfasst, das als Antwort auf die Sendung des ersten Prüfsignals erfasst wird, und ein zweites Rücksignal, das als Antwort auf die Sendung des zweiten Prüfsignals erfasst wird, wobei die Steuereinheit (2) programmiert ist, um:
- während des und mindestens eines Wartezyklus (40):
∘ das erste und zweite Rücksignal mit einem ersten und zweiten Schwellenwert zu vergleichen (431), um einen elektrischen Anschlussfehler zwischen der Vorrichtung (1) und der Steuereinheit (2) zu erkennen,
- während des und mindestens eines Aktivierungszyklus (50) nach dem und mindestens einem Wartezyklus (40):
∘ an die Vorrichtung (1) ein Aktivierungssignal zu senden (52), wenn kein elektrischer Anschlussfehler erkannt wird, wobei das Aktivierungssignal aus einem elektrischen Impulssignal besteht, das mit der ersten Frequenz (F1) gesendet wird, die aus dem Arbeitsfrequenzbereich des Wandlers (12) ausgewählt ist, wobei die elektrische Leistung des Aktivierungssignals größer ist als die elektrische Leistung der Prüfsignale,
∘ anderenfalls kein Aktivierungssignal zu senden.

5. Apparat nach einem der Ansprüche 1 bis 4, wobei der Verarbeitungsschritt einen Unterschritt umfasst, der darin besteht, die elektrische Leistung jedes Rücksignals mit mindestens eines Schwellenwert zu vergleichen.

6. Apparat nach Anspruch 1, wobei die Steuereinheit (2) programmiert ist, um:
- während des und mindestens eines Wartezyklus (40):
∘ das und mindestens eine Rücksignal zu verarbeiten (433), um einen Vibrations- oder Nicht-Vibrationszustand des Wandlers (12) zu bestimmen, um daraus einen eventuellen Anschlussfehler abzuleiten,
- während des und mindestens eines Aktivierungszyklus (50) nach dem und mindestens einem Wartezyklus (40):
∘ an die Vorrichtung (1) das Aktivierungssignal zu senden (52), wenn kein elektrischer Anschlussfehler erkannt wird, wobei das Aktivierungssignal aus einem elektrischen Impulssignal besteht, das mit der ersten Frequenz (F1) gesendet wird, die aus dem Arbeitsfrequenzbereich des Wandlers (12) ausgewählt ist, wobei die elektrische Leistung des Aktivierungssignals größer ist als die elektrische Leistung der Prüfsignale,
∘ anderenfalls kein Aktivierungssignal zu senden.

7. Apparat nach einem der Ansprüche 1 bis 6, wobei die Steuereinheit (2) einen Richtungskoppler (23) für die Erfassung des und mindestens einen Rücksignals umfasst, wobei der Richtungskoppler im Vorfeld mit einer Impedanzanpassungsschaltung (22) verbunden ist.

8. Verfahren zur Ermittlung eines Funktionsfehlers eines Apparats zur Unterstützung der Diagnose und/oder Behandlung einer Erkrankung durch die Anwendung von Ultraschall auf ein Gewebe, wobei der Apparat umfasst:
- eine zuvor implantierte Ultraschallerzeugungsvorrichtung (1),
- eine entfernte Steuereinheit (2) zum Versorgen der Vorrichtung (1) mit Strom und Bestimmen und Überwachen ihrer Betriebsparameter, wobei die Steuereinheit (2) geeignet ist, die Vorrichtung (1) während mindestens eines Aktivierungszyklus (50) derart mit Strom zu versorgen, dass die Vorrichtung (1) aktiviert ist, wobei jedem Aktivierungszyklus (50) ein Wartezyklus (40) vorausgeht,
wobei die Vorrichtung (1) und die Steuereinheit (2) über elektrische Anschlussmittel (31, 32) elektrisch verbunden sind,
**dadurch gekennzeichnet, dass** das Verfahren eine Prüfphase umfasst, die während mindestens eines Wartezyklus (40) durchgeführt wird, um einen elektrischen Anschlussfehler zu erkennen, der aus einer fehlenden elektrischen Verbindung zwischen der Vorrichtung (1) und der Steuereinheit (2) besteht, wobei die Prüfphase die folgenden Schritte umfasst:
- während des und mindestens eines Wartezyklus (40):
∘ das Senden (410) mindestens eines Prüfsignals durch die Steuereinheit (2) zu einem ersten Zeitpunkt des Wartezyklus (40), wobei das und mindestens eine Prüfsignal aus einem niederfrequenten elektrischen Impulssignal besteht, das mit einer Frequenz (F1) gesendet wird, die aus einem Arbeitsfrequenzbereich eines Wandlers (12) der Vorrichtung (1) ausgewählt ist,
∘ das Erfassen (420) mindestens eines Rücksignals durch die Steuereinheit (2) zu einem zweiten Zeitpunkt des Wartezyklus (40),
∘ das Verarbeiten (430) des und mindestens eines Rücksignals, um eine Information über die Qualität des elektrischen Anschlusses zwischen der Ultraschallvorrichtung (1) und der Steuereinheit (2) zu erhalten,
- während des und mindestens eines Aktivierungszyklus (50) nach dem und mindestens einem Wartezyklus (40) das Senden (51, 52) eines Signals in Abhängigkeit von der über die Qualität des elektrischen Anschlusses erhaltene Information, wobei das Signal besteht aus:
∘ einem Alarmsignal, wenn die Steuereinheit (2) nicht korrekt an die Ultraschallvorrichtung (1) angeschlossen ist.

9. Verfahren nach Anspruch 8, wobei das und mindestens eine Prüfsignal umfasst:
- ein erstes Prüfsignal, das aus einem elektrischen Impulssignal besteht, das mit der ersten Frequenz (F1) gesendet wird, die aus dem Arbeitsfrequenzbereich des Wandlers (12) der Vorrichtung (1) ausgewählt ist,
- ein zweites Prüfsignal, das aus einem elektrischen Impulssignal besteht, das mit einer zweiten Frequenz (F2) gesendet wird, die außerhalb des Arbeitsfrequenzbereichs des Wandlers (12) ausgewählt ist.

10. Verfahren nach Anspruch 9, das die folgenden Schritte umfasst:
- während des und mindestens eines Wartezyklus (40):
∘ Senden (411) des ersten Prüfsignals durch die Steuereinheit und Erfassen (421) eines ersten Rücksignals als Antwort durch die Steuereinheit,
∘ Senden (412) des zweiten Impulsprüfsignals durch die Steuereinheit und Erfassen (422) eines zweiten Rücksignals als Antwort durch die Steuereinheit,
∘ Verarbeiten (431) des ersten und zweiten Rücksignals, um einen eventuellen Anschlussfehler zu erkennen.

11. Verfahren nach Anspruch 8, das ferner eine Überwachungsphase umfasst, die während mindestens eines Wartezyklus (40) durchgeführt wird, um einen akustischen Kopplungsfehler zwischen der Ultraschallerzeugungsvorrichtung und dem Gewebe zu erkennen.

## Claims

1. An apparatus for diagnosis assistance and/or treatment of a pathology comprising:
- an ultrasound generator device (1), previously implanted,
- a remote control unit (2) for determining and controlling operating parameters of the ultrasound generator device (1), and providing it with electricity during at least one activation cycle (50), each activation cycle (50) being preceded by a standby cycle (40),
- means of electrical connection (31, 32) between the ultrasound generator device (1) and the control unit (2),
***characterised in that*** the control unit (2) is programmed to detect, during at least one standby cycle (40), a fault with the electrical junction consisting of an absence of electrical link between the ultrasound generator device (1) and said control unit (2), said detection of a junction fault consisting of:
- During said and at least one standby cycle (40):
∘ emitting (410, 411, 412, 413), by the control unit, at least one control signal at at least a first instant of the standby cycle, said and at least one control signal comprising a low-power electrical pulse signal emitted at a frequency (F1) selected within a range of working frequencies of a transducer (12) of the ultrasound generator device (1),
∘ acquiring (420, 421, 422, 423), by the control unit, at least one feedback signal in response to said and at least one emitted control signal,
∘ processing (430, 431, 433), by the control unit, of said and at least one feedback signal acquired to detect an electrical connection fault between the ultrasound generator device (1) and the control unit (2),
- During said and at least one activation cycle (50) subsequent to said and at least one standby cycle (40):
∘ emitting (52), by the control unit, an activation signal of the ultrasound generator device (1) if no electrical junction fault is detected,
∘ absence of emission, by the control unit, of the activation signal if an electrical junction fault is detected.

2. The apparatus according to claim 1, wherein the impedance of the ultrasound generator device (1) varies depending on the frequency of the control signal.

3. The apparatus according to any of claims 1 or 2, wherein said and at least one control signal comprises:
- a first control signal consisting of an electrical pulse signal emitted at the first frequency (F1) selected within the range of working frequencies of the transducer (12) of the ultrasound generator device (1),
- a second control signal consisting of an electrical pulse signal emitted at a second frequency (F2) selected outside the range of working frequencies of the transducer (12).

4. The apparatus according to claim 3, wherein said and at least one feedback signal comprises a first feedback signal acquired in response to the emission of the first control signal, and a second feedback signal acquired in response to the emission of the second control signal, the control unit (2) being programmed to:
- During said and at least one standby cycle (40):
∘ compare (431) the first and second feedback signals to first and second threshold values to detect an electrical junction fault between the ultrasound generator device (1) and the control unit (2).
- During said and at least one activation cycle (50) consecutive to said and at least one standby cycle (40):
∘ emit (52) to the ultrasound generator device (1) an activation signal if no electrical junction fault is detected, the activation signal consisting of an electrical pulse signal emitted at the first frequency (F1) selected within the range of working frequencies of the transducer (12), the electrical power of the activation signal being greater than the electrical power of the control signals,
∘ not emit the activation signal otherwise.

5. The apparatus according to any one of claims 1 to 4, wherein the processing step comprises a sub-step consisting in comparing the electrical power of each feedback signal with at least one threshold value.

6. The apparatus according to claim 1, wherein the control unit (2) is programmed to:
- During said and at least one standby cycle (40):
∘ process (433) said and at least one feedback signal to determine a vibratory or non-vibratory state of the transducer (12) in order to deduce therefrom a possible junction fault,
- During said and at least one activation cycle (50) consecutive to said and at least one standby cycle (40):
∘ emit (52) to the ultrasound generator device (1) the activation signal if no electrical junction fault is detected, the activation signal consisting of an electrical pulse signal emitted at the first frequency (F1) selected within the range of working frequencies of the transducer (12), the electrical power of the activation signal being greater than the electrical power of the control signals,
∘ not emit the activation signal otherwise.

7. The apparatus according to any one of claims 1 to 6, wherein the control unit (2) comprises a directional coupler (23) for acquiring said and at least one feedback signal, said directional coupler being linked upstream of an impedance matching circuit (22).

8. A method for detecting a malfunction of an apparatus for diagnosis assistance and/or treatment of a pathology by applying ultrasounds on a tissue, the apparatus comprising:
- an ultrasound generator device (1), previously implanted,
- a remote control unit (2) for providing electricity to the ultrasound generator device (1) and for determining and controlling its operating parameters, the control unit (2) being adapted to provide electricity to the ultrasound generator device (1) during at least one activation cycle (50) so as to activate said ultrasound generator device (1), each activation cycle (50) being preceded by a standby cycle (40),
the ultrasound generator device (1) and the control unit (2) being electrically joined via electrical connection means (31, 32),
**characterised in that** the method comprises a control phase implemented during at least one standby cycle (40) for detecting a fault with the electrical junction consisting of an absence of electrical link between the ultrasound generator device (1) and the control unit (2), said control phase comprising the following steps:
- During said and at least one standby cycle (40):
∘ emitting (410), by the control unit, at least one control signal at a first instant of the standby cycle (40), said and at least one control signal consisting of a low-power electrical pulse signal emitted at a frequency (F1) selected within a range of working frequencies of a transducer (12) of the ultrasound generator device (1),
∘ acquiring (420), by the control unit (2), at least one feedback signal at a second instant of the standby cycle (40),
∘ processing (430) said and at least one feedback signal to obtain information on the quality of the electrical junction between the ultrasound generator device (1) and the control unit (2),
- During said and at least one activation cycle (50), subsequent to said and at least one standby cycle (40), emitting (51, 52) a signal based on the information obtained on the quality of the electrical junction, said signal consisting of:
∘ an alarm signal if the control unit (2) is not properly joined to the ultrasound generator device (1).

9. The method according to claim 8, wherein said and at least one control signal comprises:
- a first control signal consisting of an electrical pulse signal emitted at the first frequency (F1) selected within the range of working frequencies of the transducer (12) of the ultrasound generator device (1),
- a second control signal consisting of an electrical pulse signal emitted at a second frequency (F2) selected outside the range of working frequencies of the transducer (12).

10. The method according to claim 9, which comprises the following steps:
- During said and at least one standby cycle (40):
∘ emitting (411), by the control unit, a first control signal, and acquiring (421), by the control unit, a first response feedback signal,
∘ emitting (412), by the control unit, the second pulse control signal, and acquiring (422), by the control unit, a second response feedback signal,
∘ processing (431) the first and second feedback signals to detect a possible junction fault.

11. The method according to claim 8, which further comprises a monitoring phase implemented during at least one standby cycle (40) for detecting a defect in the acoustic coupling between the ultrasound generator device and the tissue.
